# Europäisches Patentamt

(19) **European Patent Office**

**Office européen des brevets**

(11) Veröffentlichungsnummer: **0 003 774**
**B1**

(12) **EUROPÄISCHE PATENTSCHRIFT**

(45) Veröffentlichungstag der Patentschrift:
01.04.81

(51) Int. Cl.³: **C 07 C 143/55, C 07 B 11/00**

(21) Anmeldenummer: **79100357.7**

(22) Anmeldetag: **08.02.79**

(54) Verfahren zur Nitrierung von aromatischen Sulfonsäuren.

(30) Priorität: **16.02.78 CH 1698/78**
**19.01.79 CH 563/79**

(43) Veröffentlichungstag der Anmeldung:
**05.09.79 Patentblatt 79/18**

(45) Bekanntmachung des Hinweises auf die Patenterteilung:
**01.04.81 Patentblatt 81/13**

(84) Benannte Vertragsstaaten:
**BE CH DE FR GB**

(56) Entgegenhaltungen:
**GB-A-1 521 643**
**Chemical Abstracts, Band 62, Nr. 9,**
**26. April 1965**
**Columbus, Ohio, USA**
**10392 d, State Scientific Research Institute of Organic**
**Intermediates and Dyes**
**HOUBEN-WEYL «Methoden der organischen Chemie»**
**4. Auflage, Band X, Teil 1, 1971,**
**GEORG THIEME VERLAG, Stuttgart**

(73) Patentinhaber: **CIBA-GEIGY AG, Patentabteilung**
**Postfach, CH-4002 Basel (CH)**

(72) Erfinder: **Albrecht, Bernhard, Im Laig 173, CH-4463 Buus**
**(CH)**
Erfinder: **Krajnik, Paul, Gassackerweg 57,**
**CH-4402 Frenkendorf (CH)**
Erfinder: **Weiskat, Werner, Dr., Lindbuckweg 20,**
**D-7850 Lörrach (DE)**
Erfinder: **Halfter, Georg, Dr., Bandweg 1, D-7889 Wyhlen**
**(DE)**
Erfinder: **Somlo, Tibor, Dr., Florastrasse 8,**
**CH-4127 Birsfelden (CH)**
Erfinder: **Portmann, Robert, Dr., Unterer**
**Rütschetenweg 38, CH-4133 Pratteln (CH)**

Verfahren zur Nitrierung von aromatischen Sulfonsäuren

Aromatische Sulfonsäuren, wie z.B. Naphthalinmonosulfonsäuren, sind bekanntlich schwer nitrierbare Verbindungen. Ihre Nitrierung erfolgt daher üblicherweise in Gegenwart von Lösungsmitteln, z.B. von etwa 70%iger Schwefelsäure. Da in den meisten Fällen die entstehenden Nitroverbindungen zu den entsprechenden Aminen reduziert werden, was üblicherweise im neutralen Bereich erfolgt, müssen die gebildeten Nitroverbindungen aus dem Reaktionsgemisch isoliert werden, was Arbeit und Schwefelsäure enthaltende umweltbelastende Abfälle bzw. Abwässer verursacht.

So erfolgt z.B. die konventionelle Nitrierung mittels Mischsäure und wurde durch N.N. Woroshzow, «Grundlagen der Synthese von Zwischenprodukten und Farbstoffen», 1966, Akademie Verlag, Berlin, Seite 163; und A.N. Shebuev und Mitarb., USSR-Patent Nr. 165.746, beschrieben. Nach der Arbeitsweise dieser Autoren entsteht bei der Nitrierung z.B. von 1- oder 2-Naphthalinsulfonsäure mit Salpetersäure in etwa 70%iger Schwefelsäure ein Gemisch der entsprechenden isomeren Nitronaphthalinsulfonsäuren, welches neutralisiert und reduziert wird.

Die dabei erhaltenen Aminonaphthalinsulfonsäuren werden dann durch Ansäuern entweder als Isomerengemisch, oder durch fraktionierte Ausfällung einzeln isoliert. Da die Nitrierung in Schwefelsäure erfolgt und die nachfolgende Reduktion der Nitronaphthalinsulfonsäuren nur im neutralen Bereich möglich ist, muss die Schwefelsäure vom Produkt abgetrennt und die Nitronaphthalinsulfonsäuren müssen in ihre Salze überführt werden. Diese Operationen sind arbeitsintensiv, benötigen ausgedehnte apparative Einrichtungen; dies wirkt sich negativ auf die Zeit-Raum-Ausbeuten aus und es entstehen dabei grosse Mengen von festen Abfällen und umweltbelastende Abwässer. Die Nitrierung unter Verwendung von Schwefelsäure stellt sowohl ökonomisch als auch ökologisch einen schwerwiegenden Nachteil dieses Herstellverfahrens dar.

Die Nitrierung von aromatischen Verbindungen mit Salpetersäure allein ist gemäss Literaturangaben (z.B. N.N. Woroshzow oben zitiertes Buch, Seiten 116/117) im allgemeinen durch die folgenden Vorgänge stark beschränkt:

– Beim Eintritt der Nitrogruppe in den aromatischen Kern wird ein Molekül Wasser abgespalten. Dieses Wasser, das im Reaktionsgemisch bleibt, setzt die Konzentration der Salpetersäure herab. Da die Geschwindigkeitskonstante der Nitrierung von der Konzentration der Salpetersäure abhängt, verringert sich mit zunehmender Verdünnung die Reaktionsgeschwindigkeit und die Reaktion kommt bei Herabsetzung der Säurekonzentration unter einen bestimmten Wert praktisch zum Stillstand.

– Durch deren Verdünnung übt Salpetersäure auf die aromatische Verbindung eher eine oxydierende als nitrierende Wirkung aus.

Bei Verwendung von hochkonzentrierten Salpetersäuren oder bei Anwendung erhöhter Temperaturen ist dagegen die Gefahr der Bildung unerwünschter, schlag- und temperaturempfindlicher, explosiver Mischungen vorhanden.

Verdünnte Salpetersäure eignet sich dagegen noch weniger als Nitrierungsmittel, da (vgl. Houben-Weyl, Methoden der organischen Chemie, Band X/1 (1971), Seite 481) verdünnte Salpetersäure (D: 1,0–1,37) erst bei Temperaturen oberhalb 100°C nitrierend auf die meisten organischen Substanzen einwirkt, aber andererseits unter diesen Bedingugnen bereits unerwünschte Oxydationsreaktionen überwiegen. Gemäss diesem Zitat beschränkt sich die Anwendung verdünnter Salpetersäure auf die extrem leicht zu nitrierenden Substanzklassen der Phenole und Amine.

In den Zitaten der folgenden Literaturstellen: N.N. Woroshzow, erwähnte Monographie, Seite 127; A.I. Titow, Z.O. Ch. 19 (1949), Seite 517; A.I. Titow und A.N. Baryschinkowa, Z.O. Ch. 22 (1952), Seite 1335; R. Schramm und F. H. Westheimer, J. Am. Chem. Xoc. 70 (1948), Seite 1782, wird des weiteren darauf hingewiesen, dass die Nitrierung von leicht nitrierbaren aromatischen Verbindungen, wie Benzol, Phenol und dessen Homologen und Naphthalin, mit verdünnter Salpetersäure nur in Gegenwart von salpetriger Säure stattfindet.

Im Englischen Patent Nr. 1 521 643 ist ein Verfahren zur Nitrierung von Naphthalinmonosulfonsäure beschrieben, welches dadurch gekennzeichnet ist, dass man in Abwesenheit von Lösungsmitteln Naphthalinmonosulfonsäuren mit ca. 30 bis 67%iger Salpetersäure nitriert. Falls man mit Salpetersäure mit einer geringeren Konzentration als ca. 50%ig nitriert, so wird gemäss diesem Verfahren zunächst durch Abdestillieren von Salpetersäure im Vakuum die Salpetersäurekonzentration im Reaktionsgemisch so weit erhöht, bis Nitrierung stattfindet.

Dieses Verfahren, das im Labor und auf relativ kleinen Anlagen zufriedenstellend durchgeführt werden kann, bereitet auf grösseren Anlagen Schwierigkeiten, da das Nitrierungsgemisch sehr dickflüssig wird und zum Schäumen neigt, weshalb die Reaktion, zumal gegen Ende, nur sehr langsam verläuft.

Es wurde nun gefunden, dass sich alle diese Schwierigkeiten auf sehr einfache Art umgehen lassen, indem man als Reaktionsmedium niedrigprozentige Salpetersäure verwendet und die Erhöhung der Salpetersäurekonzentration in einem Fallfilmverdampfer durchführt, durch den das Nitrierungsgemisch geleitet wird. Überraschenderweise steigt dabei die Ausbeute an Nitroverbindungen und man kommt ausserdem mit kürzeren Reaktionszeiten aus. Ferner ist durch die Nitrierung in einem Fallfilmverdampfer die Sicherheit des Verfahrens erhöht, da nur der relativ geringe Anteil des Nitrierungsgemisches im Fallfilmverdampfer erhöhter Temperatur und erhöhter Salpetersäurekonzentration ausgesetzt ist. Die Reaktion kann daher gut unter Kontrolle gehalten und gegebenenfalls leicht unterbrochen werden.

Die vorliegende Erfindung betrifft somit ein Verfahren zur Nitrierung von aromatischen Sulfonsäuren unter Verwendung von 30 bis 80%iger Salpetersäure und Salpetrigsäureinhibitoren in Abwesenheit von Schwefelsäure, das dadurch gekennzeichnet ist, dass man aromatische Sulfonsäure und Salpetersäure in einer solchen Konzentration zusammengibt, dass keine Nitrierung stattfindet, anschliessend im Reaktionsgemisch die Salpetersäurekonzentration durch Abdestillieren von verdünnter Salpetersäure unter Anwendung von Vakuum derart erhöht, dass Nitrierung der aromatischen Sulfonsäure erfolgt, wobei das Reaktionsgemisch fortlaufend im Kreislauf über einen Fallfilmverdampfer geleitet wird, dessen Verdampferrohre eine um 5 bis 50°C höhere Temperatur als das Reaktionsgemisch aufweisen, so dass die Erhöhung der Salpetersäurekonzentration und die Nitrierung praktisch nur im Fallfilmverdampfer stattfinden.

Als aromatische Sulfonsäuren kommen für das erfindungsgemässe Verfahren z.B. gegebenenfalls substituierte Benzol- oder Naphthalinsulfonsäuren mit vorzugsweise 1 bis 2 Sulfonsäuregruppen in Betracht, wie z.B. Naphthalindisulfonsäuren wie Naphthalin-1,5-sulfonsäure und Benzolmonosulfonsäure vor allem aber Naphthalinmonosulfonsäuren. Vorzugsweise werden solche aromatische Sulfonsäuren nitriert, die im Reaktionsmedium bei den Reaktionsbedingungen teilweise oder insbesondere vollständig gelöst sind. Bei unlöslichen Verbindungen ist der Zusatz eines Lösungsmittels, wie z.B. Tetrachloräthan, erforderlich.

Beispielsweise wird zu den wässrigen Lösungen oder Suspensionen der aromatischen Sulfonsäuren verdünnte Salpetersäure gegeben und zwar soviel, dass in der gebildeten Mischung 15 bis 60, vorzugsweise 20 bis 40%ige Salpetersäure vorliegt. Das Molverhältnis von aromatischer Sulfonsäure zu Salpetersäure soll zu Beginn der Reaktion vorzugsweise 1:1 bis 1:5, und insbesondere 1:1,2 bis 1:2 betragen.

Dem Reaktionsgemisch werden Salpeterigsäureinhibitoren, vorteilhaft in Mengen von 2 bis 50 Mol-%, insbesondere 5 bis 15 Mol-%, bezogen auf aromatische Sulfonsäure, zugesetzt.

Erfindungsgemäss verwendbare Salpetrigsäureinhibitoren sind Verbindungen, die mit salpetriger Säure reagieren und deren Reaktionsgeschwindigkeit mit salpetriger Säure gross, mit Salpetersäure hingegen klein, vorzugsweise null, ist. Als solche kommen beispielsweise salpetersäurebeständige, freie Aminogruppen enthaltende Verbindungen in Betracht. Ihr Zusatz dient der Unterdrückung der durch salpetrige Säure katalysierten oxidativen Ringspaltung.

Besonders geeignet sind: Thioharnstoff, Harnstoffnitrat, Sulfaminsäure und vor allem Harnstoff.

Die Salpetersäurekonzentration des Reaktionsgemisches wird anschliessend im Fallfilmverdampfer durch Evakuieren, vorzugsweise auf 10 bis 100, insbesondere 20 bis 70 Torr, unter Abdestillieren von verdünnter, z.B. ca. 30 bis 67%iger Salpetersäure erhöht, wobei unter gleichzeitigem Erwärmen des Reaktionsgemisches vorzugsweise auf 40 bis 70, insbesondere 50 bis 60°C Nitrierung während der kurzen Verweilzeit im Fallfilmverdampfer stattfindet. Die Verdampferrohre im Fallfilmverdampfer weisen eine um 5 bis 50, vorzugsweise 10 bis 30°C höhere Temperatur auf als das Reaktionsgemisch. Unter diesen Bedingungen erfolgt die Verdampfung von verdünnter Salpetersäure im wesentlichen im Fallfilmverdampfer. Die Nitrierung findet in Abhängigkeit von Produkt und Temperatur mit ca. 30 bis 67%iger Salpetersäure statt.

Die Reaktionsdauer hängt von den zu nitrierenden Verbindungen und den Reaktionsbedingungen ab. Vorzugsweise beträgt sie 30 Minuten bis 24 Stunden, insbesondere 30 Minuten bis 8 Stunden.

Fallfilmverdampfer, auch Fallstromverdampfer genannt, sind allgemein bekannt. Sie bestehen im Prinzip aus Verdampferrohren, auf die die Flüssigkeit von oben mit geeigneten Vorrichtungen verteilt wird. Die Flüssigkeit strömt dann mit dem entstehenden Dampf nach unten in einen Abscheider für Destillat und Konzentrat. Solche Verdampfer sind z.B. in «Ullman's Encyklopädie der technischen Chemie», 4. Auflage, Band 2, S. 655 ff, beschrieben.

Eine bevorzugte Ausführungsform des erfindungsgemässen Verfahrens ist die folgende:

Eine 50 bis 80%ige wässerige Lösung einer aromatischen Sulfonsäure wird in einem Reaktionsgefäss mit der oben beschriebenen Menge an verdünnter Salpetersäure und Salpetrigsäureinhibitor unter Rühren vermischt. Das Reaktionsgefäss wird evakuiert und auf die obengenannte Temperatur erwärmt. Dann wird das Reaktionsgemisch im Kreislauf über einen Fallfilmverdampfer geleitet, wobei verdünnte Salpetersäure abdestilliert. Nachdem die exotherme Reaktion nach partieller Umsetzung abgeklungen ist, wird dem Reaktionsgemisch weitere Salpetersäure zugesetzt, vorzugsweise als 50 bis 70%ige Säure, bis eine Menge von 1 bis 5, insbesondere 1,5 bis 3,5 Mol Salpetersäure pro Mol aromatische Sulfonsäure erreicht ist. Zweckmässig wird die 50 bis 70%ige Salpetersäure durch Vermischen der abdestillierenden etwa 30%igen Salpetersäure mit hochkonzentrierter, 98%iger Säure erhalten.

Anschliessend wird das Reaktionsgemisch noch 1 bis 6 Stunden unter Rückfluss weiter reagieren gelassen, wobei das Reaktionsgemisch fortwährend im Kreislauf über den Fallfilmverdampfer geleitet wird.

Danach liegt das Reaktionsprodukt als Lösung bzw. Anschlämmung in einer stark verdünnten «Abfallsäure» (15 bis 40%ig) vor, die unter Vakuum (30 bis 20 Torr und 50 bis 80°C) aus dem Reaktionsgemisch abdestilliert und wieder eingesetzt werden kann.

Die erhaltenen nitrierten aromatischen Sulfonsäuren können nun auf übliche Weise isoliert werden. Vorteilhaft werden sie jedoch ohne Zwischenisolierung (mit oder ohne Abfallsäure) mit Wasser verdünnt, neutralisiert (z.B. mit Salmiakgeist, Dolomit oder Kreide) und zu den entsprechenden Aminosulfonsäuren reduziert (z.B. nach der Béchamps-Reduktion, der katalytischen Hydrierung oder der Reduktion mit Hydrazinhydrat etc.).

Die Vorteile des neuen Verfahrens liegen vor allem in der einfachen Durchführung der Nitrierung, den hohen Nitrierungsausbeuten und den guten Raum-Zeit-Ausbeuten. Darüberhinaus wird die Weiterverarbeitung der nitrierten Zwischenprodukte zu den Naphthylaminsulfonsäuren wesentlich vereinfacht, da die Nitrierung nach dem neuen Verfahren in Abwesenheit von Schwefelsäure abläuft. Es ist nicht mehr notwendig, die Schwefelsäure vor der nachfolgenden Reduktion in Form von Gips auszufällen. Nach dem vorliegenden Verfahren wird direkt nach der Nitrierung neutralisiert und zu den Naphthylaminsulfonsäuren reduziert. Durch diese Arbeitsweise werden die Abwassermengen deutlich verringert, und feste Abfälle, wie z.B. Gips, treten überhaupt nicht mehr auf. Daraus resultiert eine wesentlich verbesserte Ökologie des neuen Verfahrens im Vergleich zu den bekannten Nitrierprozessen unter Verwendung von Mischsäure.

Die Nitrosulfonsäuren und vor allem die Aminosulfonsäuren stellen wertvolle Zwischenprodukte für die Herstellung von Farbstoffen dar. So können z.B. durch Diazotierung der 1-Naphthylamin-5-sulfonsäure (Laurent-Säure) und anschliessende Kupplung mit β-Naphthol Azofarbstoffe des Typs C.I. Acid Red 141, oder durch Kupplung von 1-Naphthylamin-7-sulfonsäure (Cleve-Säure-1,7) auf Naphtionsäure und anschliessende weitere Diazotierung und Kupplung mit 1-Naphthylamin Diazofarbstoffe des Typs C.I. Acid Black 27 erhalten werden.

Die folgenden Beispiele veranschaulichen die Arbeitsweise des erfindungsgemässen Verfahrens. Darin sind die Temperaturen in Celsiusgraden angegeben, Teile bedeuten Gewichtsteile und Prozente Gewichtsprozente.

Beispiel 1:

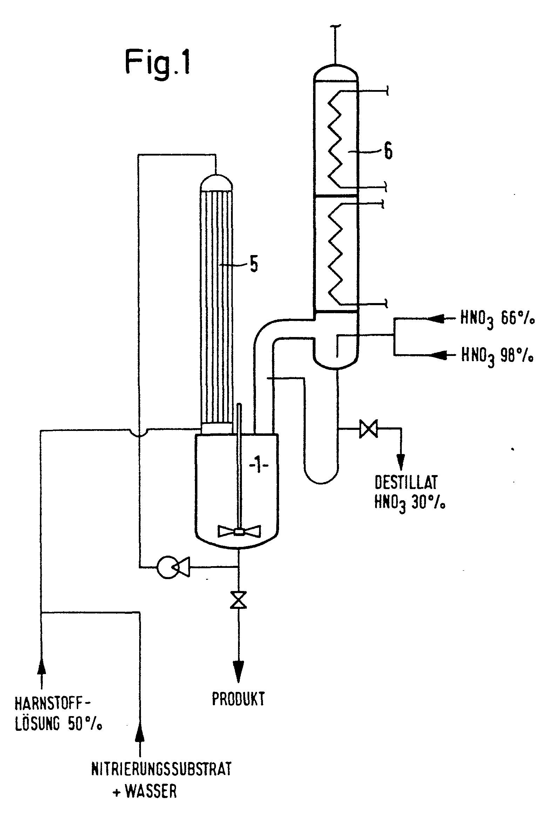

In Fig. 1 ist eine Anlage mit Fallfilmverdampfer dargestellt.

In den auf 50 Torr evakuierten Reaktor (1) werden 78 kg 70%ige wässerige Naphthalinsulfonsäurelösung (enthaltend 46,86 kg 1-Naphthalinsulfonsäure, sowie Isomere), 4,32 kg 50%ige Harnstofflösung und 32,25 kg 66%ige Salpetersäure eingespeist.

Das Reaktionsgemisch wird im Kreislauf über die auf 70°C aufgeheizten Verdampferrohre des Fallfilmverdampfers (5) geleitet und der Druck in der Anlage wird so erniedrigt, dass eine Innentemperatur im Reaktor von 50° aufrechterhalten bleibt. Innerhalb von 2 Stunden werden 40,05 kg 30%iger Salpetersäure abdestilliert.

Anschliessend wird auf Rückflussdestillation umgeschaltet und unter fortwährendem Umpumpen des Reaktionsgemisches über den Fallfilmverdampfer während einer Stunde 18,54 kg 98%ige Salpetersäure am unteren Ende des Kondensators (6) zugegeben und zwar so, dass die 98%ige Salpetersäure mit dem Rückfluss auf ca. 60%ige Salpetersäure verdünnt wird, bevor sie in den Reaktor fliesst.

Nach Beendigung der Zugabe von Salpetersäure lässt man die Reaktionslösung noch während einer Stunde weiterreagieren, wobei sie fortwährend über den Fallfilmverdampfer geleitet wird.

Die Reaktionslösung wird abgelassen, das erhaltene Nitrierungsprodukt in üblicher Weise reduziert, liefert ein Gemisch von 30,9 kg Perisäure (1-Naphthylamin-8-sulfonsäure) (61,5% der Theorie, bezogen auf eingesetzte 1-Naphthalinsulfonsäure) und 8,8 kg Laurent-Säure (1-Naphthylamin-5-sulfonsäure) 18% der Theorie.

Beispiel 2:

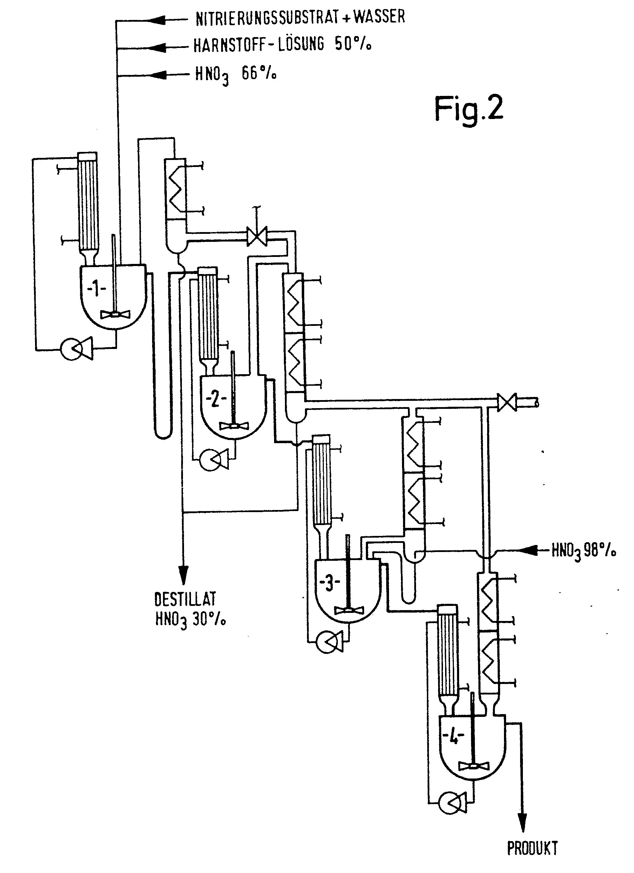

In Fig. 2 ist eine kontinuierliche Anlage zur Nitrierung von 1-Naphthalinsulfonsäure dargestellt.

In den auf 40 Torr evakuierten Reaktor (1) werden kontinuierlich die Reaktionskomponenten eingespeist und zwar im Verhältnis 374 Teile 70%ige wässrige Naphthalinsulfonsäurelösung (enthaltend 208 Teile 1-Naphthalinsulfonsäure sowie Isomere) auf 19 Teile 50%ige Harnstofflösung und 143 Teile 66%ige Salpetersäure.

Das Reaktionsgemisch wird im Kreislauf über die auf 70°C erwärmten Verdampferrohre des Fallfilmverdampfers geleitet und der Druck in der Anlage wird so erniedrigt, dass eine Innentemperatur im Reaktor von 50° aufrecht erhalten bleibt. Es destilliert kontinuierlich 30%ige Salpetersäure ab.

Das konzentriertere Reaktionsgemisch fliesst kontinuierlich via Überlauf, Siphon und Fallfilmverdampfer in den Reaktor (2), wo die weitere Einengung bei 20 Torr und 50°C erfolgt, indem wie oben beschrieben, das Reaktionsgemisch im Kreislauf über einen Fallfilmverdampfer geleitet wird. Aus den Reaktoren (1) und (2) destillieren insgesamt 172 Teile 30%ige Salpetersäure pro eingesetzte 143 Teile 66%ige Salpetersäure ab. Das Reaktionsgemisch fliesst über einen weiteren Fallfilmverdampfer in den Reaktor (3), wo, bezogen auf den obengenannten Ansatz, 82 Teile 98%ige Salpetersäure simultan am unteren Ende des Kondensators eingespeist, mit dem Kondensat verdünnt werden und als 60%ige Salpetersäure zum Reaktionsgemisch zufliessen.

Dieses fliesst über einen weiteren Fallfilmverdampfer in den Reaktor (4), wo die Nachreaktion erfolgt. Über einen Überlauf verlässt das ausreagierte Nitrierungsprodukt die Anlage als zähflüssige Masse, aus der nach Reduktion in der üblichen Weise ca. 20% Laurent- und ca. 60% Perisäure, bezogen auf eingesetzte 1-Naphthylaminsulfonsäure, gewonnen werden. Die durchschnittliche Verweilzeit in der Anlage beträgt 4 bis 6 Stunden.

Beispiel 3:

In eine Anlage gemäss Fig. 1 werden 158 g Benzolsulfonsäure, 9,6 g Harnstoff, 1 g HCl 20%ige und 200 g 63%ige Salpetersäure in den Reaktor (1) eingespeist.

Das Reaktionsgemisch wird im Kreislauf über die auf 70°C aufgeheizten Verdampferrohre des Fallfilmverdampfers (2) geleitet und der Druck in der Anlage wird so erniedrigt, dass eine Innentemperatur im Reaktor von 50°C aufrechterhalten bleibt. Innerhalb von 2 Stunden werden 175 g 44,4%ige Salpetersäure abdestilliert.

Anschliessend wird auf Rückflussdestillation umgeschaltet und unter fortwährendem Umpumpen des Reaktionsgemisches über den Fallfilmverdampfer während einer Stunde 126 g 98%ige Salpetersäure am unteren Ende des Kondensators zugegeben und zwar so, dass die 98%ige Salpetersäure mit dem Rückfluss auf ca. 60%ige Salpetersäure verdünnt wird, bevor sie in den Reaktor fliesst.

Nach Beendigung der Zugabe von Salpetersäure lässt man die Reaktionslösung noch während 3 Stunden weiterreagieren, wobei sie fortwährend über den Fallfilmverdampfer geleitet wird.

Die Reaktionslösung wird abgelassen, mit Wasser auf 500 g aufgefüllt und in einem Dünnschichtverdampfer bei 40 Torr und 73°C die Salpetersäure abgedampft.

Aus dem Rückstand werden 114,5 g (56% d. Theorie) m-Nitrobenzolsulfonsäure erhalten.

Beispiel 4:

In einer Anlage gemäss Fig. 1 werden 100 g Wasser, 9,6 g Harnstoff, 1 g HCl 35%ig, 200 g $HNO_3$ 63%ig und 384 g Armstrongsäuretetrahydrat mit folgender Zusammensetzung eingefüllt.

75 Gew.-% Naphthalin-1,5-disulfonsäure
0,1 Gew.-% Naphthalin-1,6-disulfonsäure
0,3 Gew.-% Naphthalin-1,3,5-trisulfonsäure
2,6 Gew.-% Schwefelsäure
22,0 Gew.-% Wasser

Der Druck in der Anlage wird auf 80 Torr erniedrigt und die Temperatur im Reaktor auf 65°C eingestellt. Das Reaktionsgemisch wird im Kreislauf über die auf 80 bis 85°C aufgeheizten Verdampferrohre des Fallfilmverdampfers (2) geleitet und innerhalb von 2 Stunden 244 g $HNO_3$ 27,5% abdestilliert, die Temperatur steigt dabei auf 68 bis 70°C. Anschliessend wird auf Rückflussdestillation umgeschaltet und unter fortwährendem Umpumpen des Reaktionsgemisches über den Fallfilmverdampfer innerhalb von einer Stunde 126 g 100%ige Salpetersäure am unteren Ende des Kondensators (3) zugegeben, und zwar so, dass die 100%ige Salpetersäure mit dem Rückfluss auf ca. 65%ige Salpetersäure verdünnt wird, bevor sie in den Reaktor fliesst.

Nach Beendigung der Zugabe der Salpetersäure lässt man die Reaktionslösung während 6 Stunden weiterreagieren, wobei sie fortlaufend über den Fallfilmverdampfer geleitet wird. Anschliessend wird bei einem Druck von 20 Torr und einer von 42°C auf 53°C ansteigenden Temperatur 78 g 50%ige Salpetersäure abdestilliert. Die Reaktionslösung wird abgelassen, das erhaltene Nitrierungsprodukt in üblicher Weise reduziert, liefert 147 g 1-Naphthylamin-4,8-disulfonsäure und 16 g 2-Naphthylamin-4,8-disulfonsäure. (53.8% der Theorie).

**Patentansprüche**

1. Verfahren zur Nitrierung von aromatischen Sulfonsäuren unter Verwendung von 30 bis 80%iger Salpetersäure und Salpetrigsäureinhibitoren in Abwesenheit von Schwefelsäure, dadurch gekennzeichnet, dass man aromatische Sulfonsäure und Salpetersäure in einer solchen Konzentration zusammengibt, dass keine Nitrierung stattfindet, anschliessend im Reaktionsgemisch die Salpetersäurekonzentration durch Abdestillieren von verdünnter Salpetersäure unter Anwendung von Vakuum derart erhöht, dass Nitrierung der aromatischen Sulfonsäure erfolgt, wobei das Reaktionsgemisch fortlaufend im Kreislauf über einen Fallfilmverdampfer geleitet wird, dessen Verdampferrohre eine um 5 bis 50°C höhere Temperatur als das Reaktionsgemisch aufweisen, so dass die Erhöhung der Salpetersäurekonzentration und die Nitrierung praktisch nur im Fallfilmverdampfer stattfinden.

2. Verfahren gemäss Patentanspruch 1, dadurch gekennzeichnet, dass man Benzolmonosulfonsäure oder Naphthalinmonosulfonsäure oder Naphthalin-1,5-disulfonsäure nitriert.

3. Verfahren gemäss Patentanspruch 1, dadurch gekennzeichnet, dass zu Beginn der Reaktion das Molverhältnis von aromatischer Sulfonsäure zu Salpetersäure 1:1 bis 1:5, vorzugsweise 1:1,2 bis 1:2, beträgt.

4. Verfahren gemäss Patentanspruch 1, dadurch gekennzeichnet, dass zu Beginn die Salpetersäurekonzentration 15 bis 60, vorzugsweise 20 bis 40%, beträgt.

5. Verfahren gemäss Patentanspruch 1, dadurch gekennzeichnet, dass man als Salpetrigsäureinhibitoren salpetersäurebeständige, freie Aminogruppen enthaltende Verbindungen verwendet.

6. Verfahren gemäss den Ansprüchen 1 bis 4, dadurch gekennzeichnet, dass man als Salpetrigsäureinhibitoren Thioharnstoff, Harnstoffnitrat, Sulfaminsäure oder insbesondere Harnstoff in einer Menge von 2 bis 50 Mol-%, insbesondere 5 bis 15 Mol-%, bezogen auf aromatische Sulfonsäure, zusetzt.

7. Verfahren gemäss Patentanspruch 1, dadurch gekennzeichnet, dass man die Nitrierung bei einer Temperatur von 40 bis 70°C, insbesondere 50 bis 60°C, des Reaktionsgemisches im Fallfilmverdampfer und 10 bis 100, insbesondere 20 bis 70 Torr, durchführt.

8. Verfahren gemäss Patentanspruch 1, dadurch gekennzeichnet, dass das Reaktionsge-

9 · 0 003 774 · 10

misch während 30 Minuten bis 24 Stunden im Kreislauf über den Fallfilmverdampfer geleitet wird.

9. Verfahren gemäss Patentanspruch 1, dadurch gekennzeichnet, dass man ein Gemisch aus aromatischer Sulfonsäure, Salpetersäure und Salpetrigsäureinhibitor in einem Molverhältnis von 1:1,2:0,05 bis 1:2:0,15 verwendet, das Reaktionsgemisch bei 20 bis 50 Torr auf 50 bis 60 °C erwärmt und während 30 Minuten bis 8 Stunden im Kreislauf über einen Fallfilmverdampfer leitet, dessen Verdampferrohre eine um 10 bis 30 °C höhere Temperatur aufweisen.

**Claims:**

1. A process for nitrating aromatic sulfonic acids using 30% to 80% nitric acid and nitrous acid inhibitors, in the absence of sulfuric acid, characterised in that aromatic sulfonic acid and nitric acid are mixed in such a concentration that nitration does take place, then the concentration of the nitric acid in the reaction mixture is raised by distilling off dilute nitric acid in vacuo such that the aromatic sulfonic acid is nitrated, the reaction mixture being passed continuously in successive cycles through a falling film evaporator, the evaporator tubes of which have a temperature which is between 5° and 50 °C higher than the temperature of the reaction mixture, so that the increase in the concentration of nitric acid and the nitration in effect take place only in the falling film evaporator.

2. A process according to claim 1, characterised in that benzene-monosulfonic acid or naphthalene-monosulfonic acid or naphthalene-1,5-disulfonic acid is nitrated.

3. A process according to claim 1, characterised in that the molar ratio of aromatic sulfonic acid to nitric acid at the start of the reaction is 1:1 to 1.5, preferably 1:1.2 to 1:2.

4. A process according to claim 1, characterised in that the concentration of nitric acid at the start of the reaction is 15 to 60%, preferably 20 to 40%.

5. A process according to claim 1, characterised in that the nitrous acid inhibitors are compounds which contain free amino groups and are resistant to nitric acid.

6. A process according to claims 1 to 4, characterised in that thiourea, urea nitrate, sulfamic acid or, in particular, urea, are used as nitrous acid inhibitors in an amount of 2 to 50 mol.% especially 5 to 15 mol.%, based on the aromatic sulfonic acid employed.

7. A process according to claim 1, characterised in that the nitration is carried out at a temperature between 40° and 70 °C, in particular between 50° and 60 °C, of the reaction mixture in the falling film evaporator, and at a pressure between 10 and 100 torr, especially between 20 and 70 torr.

8. A process according to claim 1, characterised in that the reaction mixture is passed in successive cycles through the falling film evaporator for 30 minutes to 24 hours.

9. A process according to claim 1, characterised in that a mixture consisting of aromatic sulfonic acid, nitric acid and nitrous acid inhibitor in a molar ratio of 1:1, 2:0.05 to 1:2:0.15 is heated at 20 to 50 torr to 50° to 60 °C and passed in successive cycles for 30 minutes to 8 hours through a falling film evaporator, the evaporator tubes of which have a temperature which is 10° to 30 °C higher than the temperature of the reaction mixture.

**Revendications**

1. Procédé pour la nitration d'acides sulfoniques aromatiques en utilisant de l'acide nitrique de 30 à 80% et des inhibiteurs d'acide nitreux en l'absence d'acide sulfurique, caractérisé par le fait qu'on ajoute ensemble l'acide sulfonique aromatique et l'acide nitrique en une concentration telle qu'aucune nitration n'a lieu, puis que dans le mélange réactionnel, on augmente la concentration de l'acide nitrique par distillation de l'acide nitrique dilué, en utilisant le vide, de façon à ce que la nitration de l'acide sulfonique aromatique s'effectue, pendant que le mélange réactionnel est continuellement recyclé sur un évaporateur à film tombant dont les tubes évaporateurs présentent une température supérieure de 5° à 50 °C à celle du mélange réactionnel, de sorte que l'augmentation de la concentration de l'acide nitrique et la nitration n'ont pratiquement lieu que dans l'évaporateur à film tombant.

2. Procédé selon la revendication 1, caractérisé par le fait qu'on nitre l'acide benzènemonosulfonique ou l'acide naphtalènemonosulfonique ou l'acide naphtalène-1,5-disulfonique.

3. Procédé selon la revendication 1, caractérisé par le fait qu'au début de la réaction le rapport molaire de l'acide sulfonique aromatique à l'acide nitrique est de 1:1 à 1:5, de préférence de 1:1,2 à 1:2.

4. Procédé selon la revendication 1, caractérisé par le fait qu'au début la concentration de l'acide nitrique est de 15 à 60%, de préférence de 20 à 40%.

5. Procédé selon la revendication 1, caractérisé par le fait qu'on utilise, comme inhibiteurs d'acide nitreux, des composés résistant à l'acide nitrique, renfermant des groupes amino.

6. Procédé selon l'une quelconque des revendications 1 à 4, caractérisé par le fait qu'on ajoute comme inhibiteurs d'acide nitreux: la thio-urée, le nitrate d'urée, l'acide sulfamique ou en particulier l'urée en une quantité de 2 à 50% en mole, en particulier de 5 à 15% en mole par rapport à l'acide sulfonique aromatique.

7. Procédé selon la revendication 1, caractérisé par le fait qu'on réalise la nitration à une température de 40° à 70 °C, en particulier de 50° à 60 °C, du mélange réactionnel dans l'évaporateur à film tombant et sous 13,3 à 133 mbars, en particulier sous 26,6 à 93,1 mbars.

8. Procédé selon la revendication 1, caractérisé par le fait que le mélange réactionnel est recyclé pendant 30 minutes à 24 heures sur l'évaporateur

6

à film tombant.

9. Procédé selon la revendication 1, caractérisé par le fait qu'on utilise un mélange d'acide sulfonique aromatique, d'acide nitrique et d'inhibiteur d'acide nitreux dans un rapport molaire de 1:1,2:0,05 à 1:2:0,5, qu'on chauffe le mélange réactionnel à 50–60°C sous 26,6 à 66,5 mbars et qu'on le recycle pendant 30 minutes à 8 heures sur un évaporateur à film tombant dont les tubes évaporateurs ont une température supérieure de 10 à 30°C

0 003 774

# Fig.1

5

6

HNO₃ 66%

HNO₃ 98%

-1-

DESTILLAT
HNO₃ 30%

HARNSTOFF-
LÖSUNG 50%

PRODUKT

NITRIERUNGSSUBSTRAT
+ WASSER

9

# 0 003 774

NITRIERUNGSSUBSTRAT + WASSER

HARNSTOFF-LÖSUNG 50%

$HNO_3$ 66%

## Fig.2

DESTILLAT
$HNO_3$ 30%

$HNO_3$ 98%

PRODUKT